# EUROPEAN PATENT SPECIFICATION

(11) **EP 4 440 559 B1**
(45) Date of publication and mention of the grant of the patent: **31.12.2025**
(21) Application number: 22817377.9
(22) Date of filing: 22.11.2022
(51) Int. Cl.: A61K 9/00

(54) **EPHEDRINE LIQUID FORMULATIONS**
FLÜSSIGE EPHEDRINFORMULIERUNGEN
FORMULATIONS LIQUIDES D'ÉPHÉDRINE

(30) Priority: 05.12.2021 US 202117542432
(43) Date of publication of application: 09.10.2024
(73) Proprietor: Fresenius Kabi Austria GmbH, 8055 Graz (AT)
(72) Inventor: UELLEN, Andreas, 8054 Graz (AT); KITZ, Kerstin, 8222 Feistritztal (AT); ZAUNER, Christoph, 8401 Kalsdorf bei Graz (AT); STEURER, Matthias, 8041 Graz (AT)
(74) Representative: Fresenius Kabi Deutschland GmbH
(86) International application number: PCT/IB2022/061283
(87) International publication number: WO 2023/100031

(56) References cited:
- CA-A- 498 033
- US-B1- 10 869 845
- US-B2- 11 090 278

## Description

### BACKGROUND OF THE INVENTION

Ephedrine is a substituted amphetamine that functions as a stimulant and is used in medicine to increase blood pressure and to induce bronchodilation. Ephedrine can be administered orally or by injection. Ephedrine exhibits optical isomerism, and the isomer that is marketed is (-)-(1R,2S)-ephedrine. Ephedrine, also referred to as (1R,2S)-2-(methylamino)-1-phenylpropan-1-ol, has a molecular weight of approximately 165.2 and the following chemical structure:

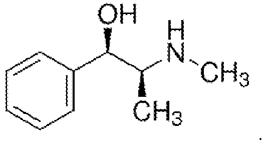

In medicine, injections of ephedrine, or a pharmaceutically acceptable salt thereof, are indicated for the treatment of clinically important hypotension occurring in the setting of anesthesia. AKOVAZ^{™} injection and CORPHEDRA^{™} injection are available in the United States as a sterile solution in a glass vial containing 50 mg/mL ephedrine sulfate (equivalent to 38 mg ephedrine base) in water for injection, with a pH of 4.5 to 7.0. The prescribing information states that these products must be diluted before administration as an intravenous bolus by withdrawing 50 mg (1 mL) of AKOVAZ^{™} injection or CORPHEDRA^{™} injection and diluting with 9 mL of 5% Dextrose Injection or 0.9% Sodium Chloride Injection. Pharmaceutical products containing 50 mg/mL of ephedrine sulfate provided in glass vials or glass ampules are available from several generic pharmaceutical manufacturers.

Another AKOVAZ^{™} injection product containing 25 mg/5 mL ephedrine sulfate supplied in a prefilled syringe was approved for use in the United States in August 2021. Each mL of the AKOVAZ^{™} prefilled syringe product contains 5 mg ephedrine sulfate (equivalent to 3.8 mg ephedrine base) and 9 mg sodium chloride, and has a pH range of 4.5 to 6.5. The prescribing information states that the AKOVAZ^{™} prefilled syringe product should not be diluted prior to use.

A pharmaceutical product containing 5 mg/mL of ephedrine sulfate was approved for use in the United States in April 2020 under the tradename EMERPHED^{™} (ephedrine sulfate) injection. EMERPHED^{™} injection is supplied as a sterile solution in a glass vial that contains 50 mg/10 mL ephedrine sulfate, equivalent to 38 mg/10mL ephedrine base. Each mL contains ephedrine sulfate, USP 5 mg (equivalent to 3.8 mg ephedrine base), 0.9% sodium chloride, USP in water for injection. The pH is adjusted with sodium hydroxide and/or glacial acetic acid if necessary. The pH range is 4.5 to 7.0. The prescribing information states that EMERPHED^{™} injection is a pre-mixed formulation that should not be diluted before administration. US Patent No. 11,090,278 discloses a method of administering ephedrine sulfate to a subject by drawing a composition comprising about 5 mg/mL ephedrine sulfate from a sterile premixed pharmaceutical product into a syringe, and injecting the composition into the subject using the syringe. The 5 mg/mL ephedrine sulfate composition according to US Patent No. 11,090,278 has an initial pH of 4.5 to 7.0 (or 5.5 to 6.5, or 5.6 to 6.2) and a pH level within 0.5 pH units of the initial pH level after storage at 25+/-2° C and 60+/-5% relative humidity for 12 months.

A pharmaceutical product containing 5 mg/mL of ephedrine sulfate was approved for use in the United States in October 2020 under New Drug Application (NDA) 213994 currently held by Endo Ventures. The Endo product is supplied as a sterile solution in a glass vial that contains 50 mg/10 mL ephedrine sulfate, equivalent to 38 mg/10mL ephedrine base. Each mL contains 5 mg ephedrine sulfate (equivalent to 3.8 mg ephedrine base) and 9 mg sodium chloride, and sodium hydroxide and/or acetic acid for pH adjustment, if necessary. The pH range is 4.5 to 7.0. The prescribing information states that this product is a ready to use formulation that should not be diluted before administration. US Patent No. 10,869,845 discloses a storage-stable, sterile ephedrine ready-to-use solution composition comprising about 5 mg/mL of ephedrine or a pharmaceutically acceptable salt thereof; about 9 mg/mL of sodium chloride; a pH adjuster comprising acetic acid; and water, wherein the initial adjusted pH of the composition is in the range of 4.6 to 4.8; wherein the pH drift of the composition is less than 0.5 after storage for 6 months at 25° C and 60% relative humidity; and wherein the composition contains about 5% or less total impurities after storage for 6 months at 25° C and 60% relative humidity as determined by High Performance Liquid Chromatography (HPLC).

A pharmaceutical product containing 47 mg/mL, 9.4 mg/mL, or 4.7 mg/mL of ephedrine hydrochloride was approved for use in the United States in June 2021 under the tradename REZIPRES^{™} (ephedrine hydrochloride) injection for intravenous use. REZIPRES^{™} injection is supplied as a sterile solution in a glass ampule that contains 47 mg/1 mL, 47 mg/5 mL, or 23.5 mg/5 mL ephedrine hydrochloride (equivalent to 38 mg, 7.7 mg, or 3.8 mg ephedrine base per mL, respectively). REZIPRES^{™} injection products have a pH range of 5.0 to 6.5 and may contain sodium hydroxide and hydrochloric acid, if necessary, to adjust pH. REZIPRES^{™} injection 9.4 mg/mL and 4.7 mg/mL also contain sodium chloride at a concentration of 6.0 mg/mL and 7.5 mg/mL, respectively. The prescribing information states that REZIPRES^{™} injection 47 mg/mL must be diluted before administration, REZIPRES^{™} injection 9.4 mg/mL can be either used as provided or it can be diluted, and REZIPRES^{™} injection 4.7 mg/mL should not be diluted prior to use.

A pharmaceutical product containing 3 mg/mL of ephedrine hydrochloride is marketed by Aguettant in Europe. The Aguettant product is supplied as a sterile solution in a plastic (polypropylene) syringe that contains 30 mg/10 mL ephedrine hydrochloride (equivalent to 24.6 mg ephedrine per 10 mL). The other ingredients are sodium chloride, citric acid monohydrate, sodium citrate and water, and may contain hydrochloric acid or sodium hydroxide for pH adjustment. The Aguettant product has a pH range of 4.5 to 5.5.

There remains a need in the art for improved injectable formulations of ephedrine which are ready-to-administer and stable, preferably following storage for long durations at room temperature.

### BRIEF SUMMARY OF THE INVENTION

The invention provides a stable, ready-to-administer formulation comprising a therapeutically effective amount of ephedrine sulfate, a tonicity agent, about 1-10 mM of a citrate buffer, and water, wherein the formulation has a pH of from about 5.2 to about 5.4, and wherein the pH drift of the formulation is less than about 0.3 pH units following storage for at least 6 months at room temperature.

The invention also provides a stable, ready-to-administer formulation comprising about 5 mg/mL ephedrine or a pharmaceutically acceptable salt thereof, about 8.6 mg/mL sodium chloride, about 5.6 mM of a citrate buffer, and water, wherein the formulation has a pH of from about 5.2 to about 5.4, and wherein the pH drift of the formulation is less than about 0.3 pH units following storage for at least about 6 months at room temperature.

### BRIEF DESCRIPTION OF THE DRAWINGS

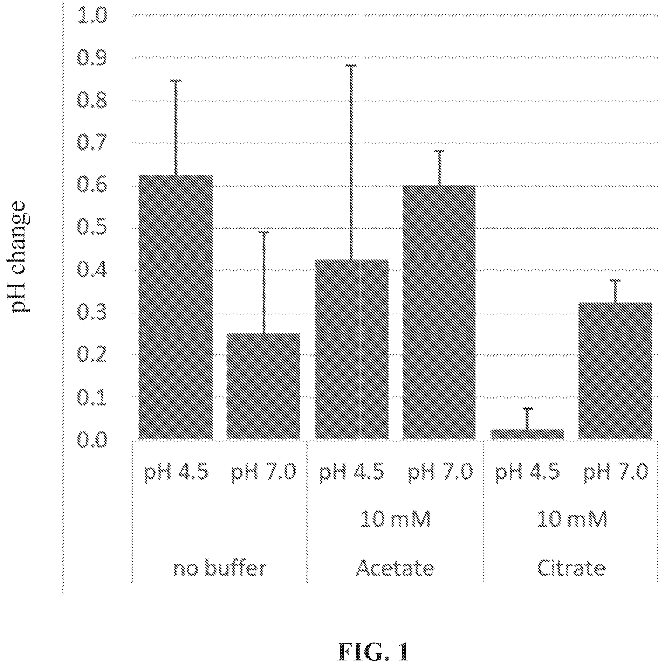

FIG. 1 is a bar graph summarizing the pH change (mean + standard deviation) of an unbuffered ephedrine formulation, an acetate-buffered formulation, or a citrate-buffered ephedrine formulation having an initial pH of about 4.5 or about 7.0 following storage for 1-month or 6-months under room temperature (25° C ±2° C / 60% RH ±5% RH) or accelerated (60° C ±2° C / 75% RH ±5% RH) conditions.

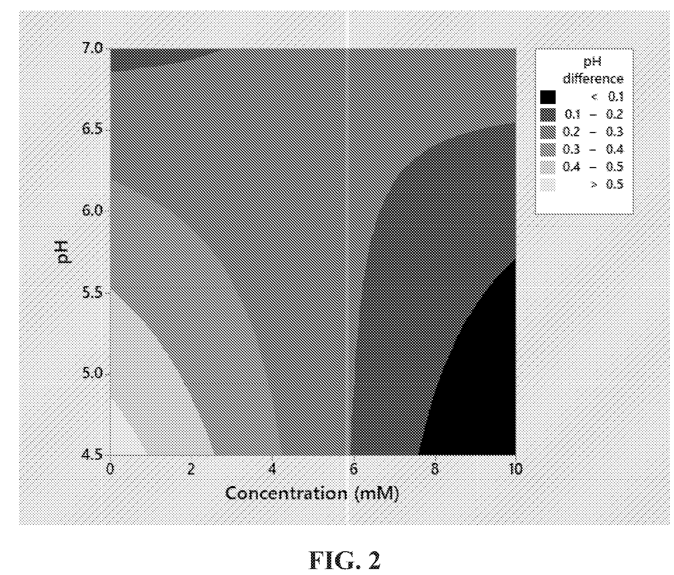

FIG. 2 shows a calculated model illustrating the pH drift as a function of citrate buffer concentration and initial pH of ephedrine formulations following storage for 6-months under room temperature (25° C ±2° C / 60% RH ±5% RH) or accelerated (42.5° C ±2° C / 75% RH ±5% RH or 60° C ±2° C / 75% RH ±5% RH) conditions.

### DETAILED DESCRIPTION OF THE INVENTION

US Patent No. 10,869,845 discloses that commercially available Ephedrine Sulfate Injection 50 mg/mL formulations have a pH of around 5.1 to 5.3, and that 5 mg/mL solutions prepared from these concentrate formulations are not pH stable and are known to have pH drifts of up to 2 pH units. US Patent No. 10,869,845 states that the inventors found that when the pH of a solution containing 5 mg/mL ephedrine sulfate, 9 mg sodium chloride, and water is set to 4.5-4.8 with acetic acid and/or sodium hydroxide, it results in a long shelf life without any pH drift, and that if the pH of the solution is greater than 4.8, a drift in pH is possible, and the pH may exceed 7.0 within the shelf life. US Patent No. 10,869,845 also discloses that a buffer could be used to stabilize the pH, but that it was found that the presence of buffers may affect the long-term stability and efficacy of ephedrine.

The present invention provides a stable, ready-to-administer formulation comprising a therapeutically effective amount of ephedrine or a pharmaceutically acceptable salt thereof, a tonicity agent, a buffer, and water, wherein the formulation has a pH of from about 5.2 to about 5.4, and wherein the pH drift of the formulation is less than about 0.3 pH units following storage for at least 6 months at room temperature.

As used herein, a "ready-to-administer" formulation refers to a sterile, injectable solution that need not be reconstituted from a solid or diluted from a concentrated solution by a healthcare provider prior to use. Rather, in the context of ephedrine formulations of the invention, a ready-to-administer formulation is supplied by a pharmaceutical manufacturer as a liquid having a pharmaceutically effective amount of ephedrine dissolved therein and contained within a suitable container (e.g., vial, syringe, bag, container, or the like) in a form that is intended to be administered to a subject without prior dilution by a healthcare provider.

The formulation according to the present invention is stable. As used herein, the terms "stable" and "stability" encompass any characteristic of the formulation which may be affected by storage conditions including, without limitation, potency, total impurities, ephedrine degradation products, specific optical rotation, optical purity, appearance, viscosity, sterility, particulates (visible and subvisible), color and/or clarity. The storage conditions which may affect stability include, for example, duration, temperature, humidity, and/or light exposure.

For example, a stable ephedrine formulation may refer to a formulation that contains at least about 90%, e.g., least about 95%, at least about 96%, or at least about 98%, of the labeled concentration of ephedrine or pharmaceutically acceptable salt thereof after storage under room temperature (e.g., 25° C ±2° C / 60% relative humidity (RH) ±5% RH) and/or accelerated (e.g., at 40° C ±2° C / 75% RH ±5% RH) conditions. A stable ephedrine formulation also may refer to a formulation that contains less than about 110%, e.g., less than about 105%, less than about 103%, or less than about 102%, of the of the labeled concentration of ephedrine or pharmaceutically acceptable salt thereof after storage under room temperature and/or accelerated conditions. A stable ephedrine formulation additionally may refer to a formulation that contains from about 95% to about 105%, from about 97% to about 103%, from about 98% to about 102%, or from about 99% to about 101%, of the labeled concentration of ephedrine or pharmaceutically acceptable salt thereof after storage under room temperature and/or accelerated conditions.

A stable ephedrine formulation also may refer to a formulation that contains less than about 5% (area percent), e.g., less than about 4% (area percent), less than about 3% (area percent), less than about 2% (area percent), or less than about 1% (area percent), of total ephedrine-related impurities present in the formulation after storage under room temperature and/or accelerated conditions. A stable ephedrine formulation additionally may refer to a formulation that contains from about 0.5% to about 5%, e.g., from about 1.5% to about 4%, from about 1.0% to about 2.5%, or from about 0.5% to about 2%, of total ephedrine-related impurities present in the formulation after storage under room temperature and/or accelerated conditions.

A stable ephedrine formulation also may refer to a formulation that contains less than about 1% (area percent), e.g., less than about 0.8% (area percent), less than about 0.4% (area percent), less than about 0.2% (area percent), or less than about 0.1% (area percent), of any individual ephedrine-related impurity present in the formulation after storage under room temperature and/or accelerated conditions. A stable ephedrine formulation additionally may refer to a formulation that contains from about 0.05% to about 0.4%, e.g., from about 0.1% to about 0.3%, from about 0.05% to about 0.2%, or from about 0.2% to about 0.6%, of any individual ephedrine-related impurity present in the formulation after storage under room temperature and/or accelerated conditions.

In some embodiments, the ready-to-administer formulation of the invention is stable for at least about 12 months, e.g., at least about 18 months, at least about 24 months, or at least about 36 months, at room temperature (e.g., at 25° C ±2° C / 60% RH ±5% RH). In other embodiments, the ready-to-administer formulation of the invention is stable for at least about 1 month, e.g., at least about 3 months, at least about 6 months, or at least about 12 months, under accelerated conditions (e.g., at 40° C ±2° C / 75% RH ±5% RH).

Methods for determining the stability of a formulation of the invention with respect to a given parameter are well-known in the art. For example, individual impurities and total impurities may be assessed by high-performance liquid chromatography (HPLC) or thin layer chromatography (TLC). Unless indicated otherwise, a percentage amount of any individual impurity or total impurities reported herein in the formulation is determined by a peak area percent method using HPLC.

In some embodiments, a stable ephedrine formulation may refer to a formulation that is colorless after storage under room temperature and/or accelerated conditions. The color of the formulation may be determined, for example, by a United States Pharmacopoeia (USP) or European Pharmacopoeia (Ph. Eur.) color method. For example, a stable ephedrine formulation of the invention may refer to a formulation that has a coloration of not less than B8 as determined by Ph. Eur. Method 2.2.2 after storage for at least 6 months at room temperature. By way of further example, a stable ephedrine formulation of the invention may refer to a formulation that has a coloration of not less than B8 as determined by Ph. Eur. Method 2.2.2 after storage for at least 1 month under accelerated conditions (e.g., at 40° C ±2° C / 75% RH ±5% RH).

The formulation may include a therapeutically effective amount of ephedrine or a pharmaceutically acceptable salt thereof such as, e.g., ephedrine sulfate or ephedrine hydrochloride. In some embodiments, the formulation of the invention includes a therapeutically effective amount of ephedrine sulfate. The amount of ephedrine or pharmaceutically acceptable salt thereof may be at a concentration of, e.g., from about 1 mg/mL to about 10 mg/mL, e.g., from about 2 mg/mL to about 8 mg/mL, or, e.g., about 5 mg/mL. In some embodiments, the formulation includes from about 2 mg/ml to about 5 mg/ml, e.g., from about 2 mg/ml to 4 mg/ml, of ephedrine base. The invention also includes embodiments in which the formulation includes about 5 mg/mL ephedrine sulfate, which corresponds to about 3.8 mg/mL ephedrine base, as well as embodiments in which the formulation includes about 3 mg/mL ephedrine hydrochloride, which corresponds to about 2.46 mg/mL ephedrine base.

The formulation may be provided in any suitable volume. In some embodiments, the volume of the formulation is about 1 mL or more, e.g., about 2 mL or more, about 3 mL or more, about 5 mL or more, about 8 mL or more, about 10 mL or more, about 20 mL or more, or about 50 mL or more. The formulation also may be provided in a volume of about 100 mL or less, e.g., about 80 mL or less, about 60 mL or less, about 40 mL or less, about 30 mL or less, about 15 mL or less, about 10 mL or less, or about 5 mL or less. The formulation additionally may be provided in a volume bounded by any two of the aforementioned endpoints. For example, the formulation may be provided in a volume of from about 1 mL to about 100 mL, e.g., from about 3 mL to about 60 mL, from about 5 mL to about 30 mL, or from about 8 mL to about 15 mL. Preferably, the formulation is provided in a volume of 3 mL to 15 mL, more preferably in a volume of 5 mL to 10 mL. In some embodiments, the volume of the formulation is about 5 mL. In other embodiments, the volume of the formulation is about 10 mL. One of ordinary skill in the art may readily select an appropriate container based upon the volume of the formulation.

The formulation of the invention may include at least one tonicity agent. Suitable tonicity agents may include, without limitation, sodium chloride, dextrose, mannitol, trehalose, potassium chloride, glycerol, and combinations thereof. In some embodiments, the tonicity agent is sodium chloride or dextrose. In some embodiments, the tonicity agent is sodium chloride. The tonicity agent may present in an amount that renders the formulation isotonic. In some embodiments, the tonicity agent is present in an amount sufficient to provide the formulation with an osmotic pressure of about 250-350 mOsm/kg, e.g., about 270-330 mOsm/kg, about 260-320 mOsm/kg, about 300-340 mOsm/kg, or about 310-330 mOsm/kg. For example, the tonicity agent may be present in an amount sufficient to provide the formulation with an osmotic pressure of about 319 mOsm/kg. The invention also includes embodiments in which the formulation includes about 8.6 mg/mL sodium chloride.

The formulation of the invention also includes at least one buffer, i.e. a citrate buffer. The type and amount of buffer present in the formulation may be selected based on several considerations, including but not limited to, a target pH, pH stabilization, impurity formation, coloration, and/or patient tolerance upon administration. In some embodiments, the buffer may include a weak acid and a conjugate base of the weak acid. The weak acid and conjugate base may be added to the formulation in an anhydrous or hydrated form. In some embodiments, the conjugate base may be present in salt form. In some embodiments, the acid or weak acid component may include a dicarboxylic acid or a tricarboxylic acid. For example, the acid component may include citric acid, isocitric acid, aconitic acid, trimesic acid, propane-1,2,3-tricarboxylic acid, fumaric acid, oxalic acid, maleic acid, malonic acid, glutaric acid, succinic acid, tartaric acid, or a combination thereof. In some embodiments, the buffer includes citric acid and a salt thereof (i.e., a citrate salt). For example, the buffer may include anhydrous citric acid and trisodium citrate dihydrate. The invention also includes embodiments in which the buffer does not include acetic acid or an acetate salt. Such embodiments include a formulation of the invention which is substantially free of acetic acid and acetate salts.

The buffer is present in an amount sufficient to provide the formulation with a pH of about 5.2-5.4. In some embodiments, the buffer is present in an amount sufficient to provide the formulation with a pH of about 5.3.

The buffer also may be present in an amount such that the formulation does not further require or include a distinct/separate pH adjusting agent, i.e., in addition to and/or distinct from the buffer. By way of example, a formulation of the invention, which includes, for example, a citrate buffer, e.g., a combination of citric acid and a citrate salt, e.g., a buffer of citric acid and sodium citrate, but does not further include a distinct/separate pH adjusting agent, would not further include or would be substantially free of a distinct or separate pH adjusting agent such as, e.g., hydrochloric acid or sodium hydroxide. One of ordinary skill in the art may readily determine the amount of buffer required to achieve the desired pH based upon the combination of a weak acid and conjugate base in the formulation. The buffer is present at a concentration of about 10 mM or less, or about 5 mM or less. The buffer also is present at a concentration of about 1 mM or more, about 2 mM or more, about 5 mM or more, about 10 mM, or about 20 mM or more. In some embodiments, the buffer is present at a concentration of about 1-10 mM, about 2-10 mM, about 4-8 mM, or about 5-6 mM. Preferably, the buffer includes a citrate buffer which is present at a concentration of about 4-8 mM, e.g., about 5-6 mM. In some embodiments, the formulation includes about 5.6 mM of a citrate buffer.

The molar ratio of buffer to ephedrine or pharmaceutically acceptable salt thereof may be about 1 or less, e.g., about 0.9 or less, about 0.8 or less, about 0.7 or less, about 0.6 or less, or about 0.5 or less. The molar ratio of buffer to ephedrine or pharmaceutically acceptable salt thereof also may be about 0.05 or more, e.g., about 0.1 or more, about 0.2 or more, about 0.3 or more, about 0.4 or more, or about 0.5 or more. The invention also includes embodiments in which the molar ratio of buffer to ephedrine or pharmaceutically acceptable salt thereof is in a range bounded by the above endpoints, e.g., wherein the ratio is in the range of about 0.05-0.9, e.g., about 0.1-0.8, about 0.2-0.6, or about 0.3-0.5. In some embodiments, the ephedrine or pharmaceutically acceptable salt thereof is ephedrine sulfate and the buffer includes a dicarboxylic acid or a tricarboxylic acid and conjugate base thereof, e.g., citric acid and sodium citrate.

The molar ratio of the weak acid component of a buffer to ephedrine or pharmaceutically acceptable salt thereof may be about 0.3 or less, e.g., about 0.25 or less, about 0.2 or less, about 0.15 or less, about 0.1 or less, or about 0.05 or less. The molar ratio of the weak acid component of a buffer to ephedrine or pharmaceutically acceptable salt thereof also may be about 0.01 or more, e.g., about 0.02 or more, about 0.04 or more, about 0.06 or more, about 0.08 or more, or about 0.1 or more. The invention also includes embodiments in which the molar ratio of the weak acid component of a buffer to ephedrine or pharmaceutically acceptable salt thereof is in a range bounded by the above endpoints, e.g., wherein the ratio is in the range of about 0.01-0.25, e.g., about 0.02-0.2, about 0.03-0.2, about 0.04-0.15, or about 0.05-0.1. In some embodiments, the ephedrine or pharmaceutically acceptable salt thereof is ephedrine sulfate and the weak acid component of a buffer includes a dicarboxylic acid or a tricarboxylic acid, e.g., citric acid.

The formulation of the invention advantageously exhibits pH stability as evidenced by low pH drift following storage under room temperature and/or accelerated conditions. In some embodiments, the pH drift of the formulation is less than about 0.3 pH units, e.g., less than about 0.25 pH units, less than about 0.2 pH units, less than about 0.15 pH units, less than about 0.1 pH units, or less than about 0.05 pH units, following storage for at least 6 months at room temperature. The invention also includes embodiments in which the pH drift of the formulation is less than about 0.3 pH units, e.g., less than about 0.25 pH units, less than about 0.2 pH units, less than about 0.15 pH units, less than about 0.1 pH units, or less than about 0.05 pH units, following storage for at least 12 months at room temperature. For example, the invention includes embodiments in which the pH drift of the formulation is less than about 0.3 pH units, e.g., less than about 0.25 pH units, less than about 0.2 pH units, less than about 0.15 pH units, less than about 0.1 pH units, or less than about 0.05 pH units, following storage for at least 24 months at room temperature.

The invention additionally includes embodiments in which the pH drift of the formulation is less than about 0.3 pH units, e.g., less than about 0.25 pH units, less than about 0.2 pH units, less than about 0.15 pH units, less than about 0.1 pH units, or less than about 0.05 pH units, following storage for at least 1 month under accelerated conditions. For instance, the invention includes embodiments in which the pH drift of the formulation is less than about 0.3 pH units, e.g., less than about 0.25 pH units, less than about 0.2 pH units, less than about 0.15 pH units, less than about 0.1 pH units, or less than about 0.05 pH units, following storage for at least 3 months under accelerated conditions. The invention also includes embodiments in which the pH drift of the formulation is less than about 0.3 pH units, e.g., less than about 0.25 pH units, less than about 0.2 pH units, less than about 0.15 pH units, less than about 0.1 pH units, or less than about 0.05 pH units, following storage for at least 6 months under accelerated conditions.

The invention accordingly provides, for example, a stable, ready-to-administer formulation comprising about 5 mg/mL ephedrine or a pharmaceutically acceptable salt thereof, a tonicity agent present in an amount sufficient to provide the formulation with an osmotic pressure of about 310-330 mOsm/kg, a buffer present in an amount sufficient to provide the formulation with a pH of from about 5.2 to about 5.4, and water, wherein the pH drift of the formulation is less than about 0.3 pH units following storage for at least about 6 months at room temperature, e.g., at least about 12 months at room temperature, e.g., at least about 24 months at room temperature.

The invention also provides, for example, a stable, ready-to-administer formulation comprising about 5 mg/mL ephedrine or a pharmaceutically acceptable salt thereof, a tonicity agent present in an amount sufficient to provide the formulation with an osmotic pressure of about 310-330 mOsm/kg, about 1-20 mM of buffer comprising a dicarboxylic acid or a tricarboxylic acid, and water, wherein the formulation has a pH of from about 5.2 to about 5.4, and wherein the pH drift of the formulation is less than about 0.3 pH units following storage for at least about 6 months at room temperature, , e.g., at least 12 months at room temperature, e.g., at least about 24 months at room temperature.

The invention additionally provides, e.g., a stable, ready-to-administer formulation comprising about 5 mg/mL ephedrine or a pharmaceutically acceptable salt thereof, about 8.6 mg/mL sodium chloride, about 5.6 mM of a citrate buffer, and water, wherein the formulation has a pH of from about 5.2 to about 5.4, wherein the pH drift of the formulation is less than about 0.3 pH units following storage for at least about 6 months at room temperature, e.g., at least about 12 months at room temperature, ,e.g., at least about 24 months at room temperature.

The invention further provides, e.g., a stable, ready-to-administer formulation consisting essentially of about 5 mg/mL ephedrine or a pharmaceutically acceptable salt thereof, a tonicity agent present in an amount sufficient to provide the formulation with an osmotic pressure of about 310-330 mOsm/kg, a buffer present in an amount sufficient to provide the formulation with a pH of from about 5.2 to about 5.4, and water, wherein the pH drift of the formulation is less than about 0.3 pH units following storage for at least about 6 months at room temperature, e.g., at least about 12 months at room temperature, e.g., at least about 24 months at room temperature.

The invention moreover provides, e.g., a stable, ready-to-administer formulation consisting essentially of about 5 mg/mL ephedrine or a pharmaceutically acceptable salt thereof, a tonicity agent present in an amount sufficient to provide the formulation with an osmotic pressure of about 310-330 mOsm/kg, about 1-20 mM of buffer comprising a dicarboxylic acid or a tricarboxylic acid, and water, wherein the formulation has a pH of from about 5.2 to about 5.4, and wherein the pH drift of the formulation is less than about 0.3 pH units following storage for at least about 6 months at room temperature, e.g., at least about 12 months at room temperature, e.g., at least about 24 months at room temperature.

The invention also provides, e.g., a stable, ready-to-administer formulation consisting essentially of about 5 mg/mL ephedrine or a pharmaceutically acceptable salt thereof, about 8.6 mg/mL sodium chloride, about 5.6 mM of a citrate buffer, and water, wherein the formulation has a pH of from about 5.2 to about 5.4, wherein the pH drift of the formulation is less than about 0.3 pH units following storage for at least about 6 months at room temperature, e.g., at least about 12 months at room temperature, e.g., at least about 24 months at room temperature.

The stable, ready-to-administer formulation of the invention that comprises, or consists essentially of, a therapeutically effective amount of ephedrine or a pharmaceutically acceptable salt thereof, a tonicity agent, a buffer, and water, having a pH of from about 5.2 to about 5.4, may further include one or more other substances. Non-limiting examples of such other substances may include, for example, one or more diluents, salts, stabilizers, solubilizers, antioxidants, preservatives, and the like, and combinations thereof.

The stable, ready-to-administer formulation of the invention that comprises, or consists essentially of, a therapeutically effective amount of ephedrine or a pharmaceutically acceptable salt thereof, a tonicity agent, a buffer, and water, having a pH of from about 5.2 to about 5.4 has excellent storage stability when stored in a pharmaceutical container. In some embodiments, the container includes a syringe, a cartridge, a vial, an ampoule, a bag, or a bottle. Preferably, the container includes a syringe or a vial.

The container may include a material such as, e.g., glass or a synthetic polymer. The synthetic polymer may include an organic polymer such as, for example, an organic polymer which includes a polyethylene, a polypropylene, a cyclic olefin polymer (COP), or a cyclic olefin copolymer (COC). In some embodiments, the container includes COC. One or more surfaces of the container may be treated with a compound, e.g., to limit reactivity with one or more components of the formulation of the invention. For example, the container may be treated with a silicone. By way of further example, the container may be treated with a sulfur-containing compound, e.g., ammonium sulfate. However, the invention also includes embodiments in which the container is not treated.

In some embodiments, the container includes a syringe. The syringe barrel may include or be made of, e.g., glass or plastic. A suitable plastic syringe may include a syringe barrel which includes or is made of an organic polymer such as, e.g., a polyethylene, a polypropylene, COP, or COC. In some embodiments, the plastic syringe barrel may include COC. The invention also includes embodiments in which the plastic syringe barrel includes an amorphous COC that is copolymerized from norbornene and ethylene using a metallocene catalyst, such as, e.g., TOPAS^{™} COC manufactured by Topas Advanced Polymers GmbH. Non-limiting examples of syringes suitable for use in the present invention are described in US Patent Application Publication No. 2015/0273133.

The invention also includes embodiments in which the container includes a vial. The vial may be made of any suitable material, which may include, for example, glass or plastic. The glass vial may include, e.g., a transparent glass vial or a light protective glass vial.

The pharmaceutical container may be sealed, e.g., by way of a closure, such as, e.g., a stopper, valve, plunger, and/or tip cap. In some embodiments, the closure may include an inert material such as, e.g., rubber or plastic. The closure also may be coated, e.g., with a silicone polymer or a fluoropolymer. However, the invention also includes embodiments which include a container in which the closure is not coated. Not limiting examples of materials that may be used in suitable closures include, for example, bromobutyl rubber, chlorobutyl rubber, and coated versions thereof.

The invention accordingly provides, for example, a syringe that includes the stable, ready-to-administer ephedrine formulation described herein, wherein the syringe barrel includes a cyclic olefin copolymer, and the plunger includes uncoated bromobutyl rubber.

A stable, ready-to-administer formulation which includes a therapeutically effective amount of ephedrine or a pharmaceutically acceptable salt thereof, a tonicity agent, a citrate buffer, and water, wherein the formulation has a pH of from about 5.2 to about 5.4, and wherein the pH drift of the formulation is less than about 0.3 pH units following storage for at least about 6 months at room temperature, may be prepared by any suitable technique, many of which are known in the art. The formulation may be prepared, e.g., in a batch or continuous process. In some embodiments, the formulation may be prepared by combining the components thereof in any order. The term "component" as used herein includes individual ingredients (e.g., ephedrine or pharmaceutically acceptable salt thereof, tonicity agent, buffer, or water, etc.) as well as any combination of ingredients (e.g., ephedrine or pharmaceutically acceptable salt thereof, tonicity agent, buffer, and/or water, etc.). In some embodiments, the formulation may be formed by combining the components together in a vessel. Such components may be combined in any order. The vessel may be made of any suitable material. In some embodiments, the vessel is made of or includes stainless steel. The manufacturing process may incorporate light protection and/or oxygen protection using methods known in the art. In some embodiments, the formulation may be prepared without light protection. The invention also includes embodiments in which the formulation is prepared without oxygen protection, and embodiments in which the formulation is prepared without light protection or oxygen protection.

In some embodiments, an amount of water equal to at least about 85% of the desired batch volume is added to a suitable vessel, then the tonicity agent and buffer are added, either sequentially or together, and the mixture is stirred until dissolution is complete. Subsequently, the ephedrine or pharmaceutically acceptable salt thereof is added, and the mixture is stirred until dissolution is complete. Next, the pH is checked and confirmed to be within the range of about 5.2-5.4. An acid (e.g., acetic acid) and/or a base (e.g., sodium hydroxide) may be added to adjust the pH to about 5.2-5.4. Alternatively, an acid or base pH adjuster is not added to the formulation. Next, an amount of water is added to bring the solution to the final desired batch volume. Optional ingredients, such as, e.g., one or more diluents, salts, stabilizers, solubilizers, antioxidants, and preservatives, may be provided to the formulation at any stage in its preparation.

The invention also includes embodiments in which the formulation is filtered, e.g., through one or more filters prior to filling the composition into one or more suitable containers, such as, e.g., a syringe, a vial, an ampoule, a cartridge, or a bag. In some embodiments, one or more filtration steps and the filling step are performed under aseptic conditions.

The filled containers may be subjected to a terminal sterilization process, such as, e.g., thermal sterilization. For example, thermal sterilization may be performed using water as a sterilizing medium. For sterilizing with water as the sterilization medium, the temperature of the water is preferably at least about 100°C, e.g., at least about 110°C, e.g., at least about 120°C. The thermal sterilization may be performed at a pressure of at least about 1 bar (100 kilopascal), for example, at least about 1.5 bar (150 kilopascal), at least about 1.7 bar (170 kilopascal), at least about 2 bar (200 kilopascal), at least about 3 bar (300 kilopascal), or at least about 4 bar (400 kilopascal). In some embodiments, thermal sterilization is performed at a pressure of from about 1 bar to about 4 bar, e.g., about 1-3 bar, about 1.5-2 bar, about 1.7-2 bar, or about 1.7-3 bar.

Thermal sterilization is may be carried out for at least about 10 minutes, e.g., for at least about 15 minutes, or for at least about 20 minutes.

In some embodiments, thermal sterilization of containers containing the stable, ready-to-administer ephedrine formulation of the invention is carried out at a temperature of about 120°C - 122°C and a pressure of about 2 bar (200 kilopascal) for about 15-20 minutes, for example, at a temperature of about 121°C and a pressure of about 2 bar (200 kilopascal) for about 15 minutes.

The formulation according to the invention is suitable for administration to a subject to treat or prevent a disease or condition, including a disease or condition that is treatable with ephedrine or a pharmaceutically acceptable salt thereof. Preferably, the subject is a mammal such as, for example, a human. The disease or condition that is treatable by the administration of ephedrine or a pharmaceutically acceptable salt thereof may include, for example, hypotension. In some embodiments, the condition may include clinically important hypotension occurring in the setting of anesthesia. The invention thus provides, for example, a method for treating hypotension including, e.g., hypotension resulting from and/or occurring during spinal or epidural anesthesia or during general anesthesia, with or without a reduction in the heart rate, by administering a formulation as described herein to a patient in need thereof. In accordance with the invention, the formulation may be administered, e.g., for a surgical and/or obstetric procedure.

### EXAMPLES

The following examples further illustrate the invention but, of course, should not be construed as in any way limiting its scope.

### EXAMPLE 1

This example demonstrates the pH drift of formulations comprising a therapeutically effective amount of ephedrine or a pharmaceutically acceptable salt thereof, a tonicity agent, a buffer, and water as a function of the buffer type.

Formulations containing 5 mg/mL ephedrine sulfate, 8.6 mg/mL sodium chloride, and either no buffer, 10 mM acetate buffer, or 10 mM citrate buffer were prepared in a stainless steel vessel, adjusted to pH of 4.5 or 7.0 with hydrochloric acid and/or sodium hydroxide, filtered, filled into plastic COC syringes, thermally sterilized, and placed into a stability chamber under room temperature (25° C ±2° C / 60% RH ±5% RH) or accelerated (60° C ±2° C / 75% RH ±5% RH) conditions. The pH of each formulation was determined after 1-month and 6-months of storage, and the results are shown in FIG. 1.

Additional formulations containing 5 mg/mL ephedrine sulfate, 8.6 mg/mL sodium chloride, and either 5 mM acetate buffer or 5 mM citrate buffer were prepared in a stainless steel vessel, adjusted to pH of 5.75 with hydrochloric acid and/or sodium hydroxide, filtered, filled into plastic COC syringes, thermally sterilized, and placed into a stability chamber under accelerated (42.5° C ±2° C / 75% RH ±5% RH) conditions. Following 3.5 months of storage, the average pH drift of 5 mM citrate-buffered formulation samples (n=5) was 0.0 and the average pH drift of 5 mM acetate-buffered formulation samples (n=5) was 0.6.

The results of this example demonstrate that citrate buffer is superior to acetate buffer in stabilizing the pH of ephedrine formulations.

### EXAMPLE 2

This example demonstrates the pH drift of exemplary formulations comprising a therapeutically effective amount of ephedrine or a pharmaceutically acceptable salt thereof, a tonicity agent, a buffer, and water as a function of the pH and buffer concentration of the formulation.

Formulations containing 5 mg/mL ephedrine sulfate, 8.0-9.0 mg/mL sodium chloride, and 0-10 mM of a citrate buffer comprising citric acid and anhydrous and trisodium citrate dihydrate were prepared, adjusted to pH of 4.5-7.0 with hydrochloric acid and/or sodium hydroxide, filtered, filled into plastic syringes, thermally sterilized, and placed into a stability chamber under accelerated conditions. The pH of each formulation was determined after 6-months of storage under room temperature (25° C ±2° C / 60% RH ±5% RH) or accelerated (42.5° C ±2° C / 75% RH ±5% RH or 60° C ±2° C / 75% RH ±5% RH) conditions, and the results are modeled in FIG. 2. In this study, the storage condition did not substantially affect the pH value and, therefore, the calculated model is expected to be applicable for all temperatures between 25 °C and 60 °C.

The results of this example demonstrate that ephedrine formulations having a pH of about 5.0 to about 5.5 have a pH drift of less than about 0.4 pH units with at least 2 mM of a buffer, less than about 0.3 pH units with at least 4 mM of a buffer, less than about 0.2 pH units with at least 6 mM of a buffer, and less than about 0.1 pH units with at least 8 mM of a buffer, following 6-months of storage under accelerated conditions.

### EXAMPLE 3

This example demonstrates the stability of exemplary formulations comprising a therapeutically effective amount of ephedrine or a pharmaceutically acceptable salt thereof, a tonicity agent, a buffer, and water as a function of container and closure types.

Formulations containing 5 mg/mL ephedrine sulfate, 7.0 mg/mL sodium chloride, and 13.7 mM of a citrate buffer comprising citric acid anhydrous and trisodium citrate dihydrate were prepared without a pH adjuster and had a native pH of about 5.2. The formulations were filtered, filled into plastic COC syringes or COP syringes, sealed with uncoated bromobutyl rubber stoppers, thermally sterilized, and placed into stability chambers under room temperature conditions (25° C ±2° C / 60% RH ±5% RH).

Following storage for 6 months, the formulations were analyzed by HPLC for individual and total impurities. The HPLC conditions were as follows:
Mobile Phase A: 200 mM ammonium acetate
Mobile Phase B: methanol
Solvent: Mobile Phase A: Mobile Phase (95:5)
Sample Temperature: 8 °C
Column: Phenyl-Hexyl (e.g., Agilent ZORBAX^{™} Eclipse Plus Phenyl-Hexyl; 250 x 4.6 mm; 3.5 µm or Waters XSELECT^{™} CSH Phenyl-Hexyl; 250 x 4.6 mm; 5 µm)
Column Temperature: 30 °C
Injection Volume: 20-25 µL
Flow Rate: 1.5-2.0 mL/min
Detection: 257 nm (ref 360 nm)
Gradient:

| Time (min) | Mobile Phase A (%) | Mobile Phase B (%) |
|---|---|---|
| 0 | 95.0 | 5.0 |
| 10.0 | 95.0 | 5.0 |
| 14.0 | 74.0 | 26.0 |
| 20.0 | 60.0 | 40.0 |
| 30.0 | 15.0 | 85.0 |
| 32.0 | 15.0 | 85.0 |
| 32.1 | 95.0 | 5.0 |
| 37.0 | 95.0 | 5.0 |

The results for individual impurities at relative retention time (RRT) 1.7, RRT 1.8, RRT 1.9, and total impurities are summarized in Table 1.

**Table 1**

| | RRT 1.7 | RRT 1.8 | RRT 1.9 | Total Imp. |
|---|---|---|---|---|
| COP | 0.43 | 0.05 | <LOQ* | 0.48 |
| COC | <LOQ | <LOQ | 0.15 | 0.15 |

| | | | | |
|---|---|---|---|---|
| * value below the limit of quantitation (LOQ) | | | | |

The results of this example demonstrate reduced individual and total impurities were detected in ephedrine formulations stored in a COC syringe as compared to a COP syringe.

### EXAMPLE 4

This example demonstrates the stability of an exemplary formulation according to the invention.

A formulation containing 5 mg/mL ephedrine sulfate, 8.6 mg/mL sodium chloride, 1.25 mg/mL trisodium citrate dihydrate and 0.25 mg/mL citric acid, anhydrous (5.6 mM of citrate buffer) was prepared in a stainless steel vessel. The formulation did not include a pH adjuster and had a native pH of about 5.3. The formulation was filtered, filled into plastic COC syringes, sealed with an uncoated bromobutyl rubber stopper, thermally sterilized, and placed into stability chambers under room temperature (25° C ±2° C / 60% RH ±5% RH) or accelerated (40° C ±2° C / 75% RH ±5% RH) conditions.

Following storage for 3 or 6 months, the pH was determined by standard technique, API assay and total impurities were determined by HPLC as described in Example 3, and coloration was determined by Ph. Eur. Method 2.2.2. The results of these analyses are summarized in Table 2.

**Table 2**

| | | **25° C / 60% RH** | | **40° C / 75% RH** | |
|---|---|---|---|---|---|
| | **t=0** | **3-mo.** | **6-mo.** | **3-mo.** | **6-mo.** |
| **pH** | 5.4 | 5.4 | 5.4 | 5.4 | 5.4 |
| **API assay** | 101% | 100% | 101% | 99% | 100% |
| **Total impurities** | <LOQ | <LOQ | <LOQ | <LOQ | <LOQ |
| **Coloration** | >B9 | >B9 | >B9 | >B9 | >B9 |

The results of this example demonstrate an aqueous ephedrine formulation comprising a tonicity agent and a citrate buffer having a pH of 5.2 to 5.4 that demonstrates excellent storage stability over at least 6 months at room temperature or 40 °C.

The use of the terms "a" and "an" and "the" and "at least one" and similar referents in the context of describing the invention (especially in the context of the following claims) are to be construed to cover both the singular and the plural, unless otherwise indicated herein or clearly contradicted by context. The use of the term "at least one" followed by a list of one or more items (for example, "at least one of A and B") is to be construed to mean one item selected from the listed items (A or B) or any combination of two or more of the listed items (A and B), unless otherwise indicated herein or clearly contradicted by context. The terms "comprising," "having," "including," and "containing" are to be construed as open-ended terms (i.e., meaning "including, but not limited to,") unless otherwise noted. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

Preferred embodiments of this invention are described herein, including the best mode known to the inventors for carrying out the invention. Variations of those preferred embodiments may become apparent to those of ordinary skill in the art upon reading the foregoing description. The inventors expect skilled artisans to employ such variations as appropriate, and the inventors intend for the invention to be practiced otherwise than as specifically described herein. Moreover, any combination of the above-described elements in all possible variations thereof is encompassed by the invention unless otherwise indicated herein or otherwise clearly contradicted by context.

## Claims

1. A stable, ready-to-administer formulation comprising a therapeutically effective amount of ephedrine sulfate, a tonicity agent, about 1-10 mM of a citrate buffer, and water, wherein the formulation has a pH of from about 5.2 to about 5.4, and wherein the pH drift of the formulation is less than about 0.3 pH units following storage for at least about 6 months at room temperature.

2. The formulation of claim 1, wherein the ephedrine sulfate is present at a concentration of about 5 mg/mL.

3. The formulation of claim 1, wherein the tonicity agent comprises sodium chloride, dextrose, mannitol, trehalose, potassium chloride, glycerol, or a combination thereof.

4. The formulation of claim 3, wherein the tonicity agent is present in an amount sufficient to provide the formulation with an osmotic pressure of about 310-330 mOsm/kg.

5. The formulation of claim 1, wherein the formulation is substantially free of a pH adjuster which is separate or distinct from the citrate buffer.

6. The formulation of claim 1, wherein the formulation has a pH drift of less than about 0.2 pH units following storage for at least about 6 months at room temperature.

7. The formulation of claim 1, wherein the formulation contains not more than about 0.2% of any individual impurity and not more than about 2% total impurities as determined by a peak area percent method by high-performance liquid chromatography (HPLC) after storage for at least about 6 months at room temperature.

8. The formulation of claim 1, wherein the formulation has a coloration of not less than B8 as determined by Ph. Eur. Method 2.2.2 after storage for at least about 6 months at room temperature.

9. A syringe comprising the formulation of claim 1, wherein the syringe barrel comprises cyclic olefin copolymer and the stopper comprises uncoated bromobutyl rubber.

10. The formulation of claim 1 comprising about 5 mg/mL ephedrine sulfate, the tonicity agent present in an amount sufficient to provide the formulation with an osmotic pressure of about 310-330 mOsm/kg, the citrate buffer present in an amount sufficient to provide the formulation with the pH of from about 5.2 to about 5.4, wherein the buffer is the citrate buffer present at a concentration of about 4-10 mM.

11. The formulation of claim 10, wherein the formulation is substantially free of a pH adjuster which is separate or distinct from the citrate buffer.

12. The formulation of claim 10, wherein the formulation has a pH drift of less than about 0.2 pH units following storage for at least about 6 months at room temperature.

13. The formulation of claim 10, wherein the formulation contains not more than about 0.2% of any individual impurity and not more than about 2% total impurities as determined by a peak area percent method by high-performance liquid chromatography (HPLC) after storage for at least about 6 months at room temperature.

14. The formulation of claim 11, wherein the formulation has a coloration of not less than B8 as determined by Ph. Eur. Method 2.2.2 after storage for at least about 6 months at room temperature.

15. A syringe comprising the formulation of claim 10, wherein the syringe barrel comprises cyclic olefin copolymer and the stopper comprises uncoated bromobutyl rubber.

## Patentansprüche

1. Stabile, verabreichungsfertige Formulierung, die eine therapeutisch wirksame Menge Ephedrinsulfat, ein Tonizitätsmittel, etwa 1-10 mM eines Citratpuffers und Wasser umfasst, wobei die Formulierung einen pH-Wert von etwa 5,2 bis etwa 5,4 aufweist und wobei die pH-Abweichung der Formulierung weniger als etwa 0,3 pH-Einheiten nach Lagerung für mindestens etwa 6 Monate bei Raumtemperatur beträgt.

2. Formulierung nach Anspruch 1, wobei das Ephedrinsulfat in einer Konzentration von etwa 5 mg/ml vorliegt.

3. Formulierung nach Anspruch 1, wobei das Tonizitätsmittel Natriumchlorid, Dextrose, Mannit, Trehalose, Kaliumchlorid, Glycerin oder eine Kombination davon umfasst.

4. Formulierung nach Anspruch 3, wobei das Tonizitätsmittel in einer Menge vorhanden ist, die ausreicht, um die Formulierung mit einem osmotischen Druck von etwa 310-330 mOsm/kg zu versehen.

5. Formulierung nach Anspruch 1, wobei die Formulierung im Wesentlichen frei von einem pH-Einsteller ist, der vom Citratpuffer getrennt oder verschieden ist.

6. Formulierung nach Anspruch 1, wobei die Formulierung eine pH-Abweichung von weniger als etwa 0,2 pH-Einheiten nach Lagerung für mindestens etwa 6 Monate bei Raumtemperatur aufweist.

7. Formulierung nach Anspruch 1, wobei die Formulierung nicht mehr als etwa 0,2 % einer einzelnen Verunreinigung und nicht mehr als etwa 2 % Gesamtverunreinigungen enthält, bestimmt durch ein Peakflächenprozentverfahren mittels Hochleistungsflüssigkeitschromatographie (HPLC) nach Lagerung für mindestens etwa 6 Monate bei Raumtemperatur.

8. Formulierung nach Anspruch 1, wobei die Formulierung eine Färbung von nicht weniger als B8, bestimmt durch Ph. Eur. Methode 2.2.2, nach einer Lagerung von mindestens etwa 6 Monaten bei Raumtemperatur aufweist.

9. Spritze, die die Formulierung nach Anspruch 1 umfasst, wobei der Spritzenzylinder zyklisches Olefin-Copolymer umfasst und der Stopfen unbeschichteten Brombutylkautschuk umfasst.

10. Formulierung nach Anspruch 1, die etwa 5 mg/ml Ephedrinsulfat, das Tonizitätsmittel in einer Menge, die ausreicht, um die Formulierung mit einem osmotischen Druck von etwa 310-330 mOsm/kg zu versehen, den Citratpuffer in einer Menge, die ausreicht, um die Formulierung mit einem pH-Wert von etwa 5,2 bis etwa 5,4 zu versehen, umfasst, wobei der Puffer der Citratpuffer ist, der in einer Konzentration von etwa 4-10 mM vorliegt.

11. Formulierung nach Anspruch 10, wobei die Formulierung im Wesentlichen frei von einem pH-Einsteller ist, der vom Citratpuffer getrennt oder verschieden ist.

12. Formulierung nach Anspruch 10, wobei die Formulierung eine pH-Abweichung von weniger als etwa 0,2 pH-Einheiten nach Lagerung für mindestens etwa 6 Monate bei Raumtemperatur aufweist.

13. Formulierung nach Anspruch 10, wobei die Formulierung nicht mehr als etwa 0,2 % einer einzelnen Verunreinigung und nicht mehr als etwa 2 % Gesamtverunreinigungen enthält, bestimmt durch ein Peakflächenprozentverfahren mittels Hochleistungsflüssigkeitschromatographie (HPLC) nach Lagerung für mindestens etwa 6 Monate bei Raumtemperatur.

14. Formulierung nach Anspruch 11, wobei die Formulierung eine Färbung von nicht weniger als B8, bestimmt durch Ph. Eur. Methode 2.2.2, nach einer Lagerung von mindestens etwa 6 Monaten bei Raumtemperatur aufweist.

15. Spritze, die die Formulierung nach Anspruch 10 umfasst, wobei der Spritzenzylinder zyklisches Olefin-Copolymer umfasst und der Stopfen unbeschichteten Brombutylkautschuk umfasst.

## Revendications

1. Formulation stable prête à être administrée comprenant une quantité thérapeutiquement efficace de sulfate d'éphédrine, un agent de tonicité, environ 1 à 10 mM d'un tampon citrate, et de l'eau, dans laquelle la formulation a un pH allant d'environ 5,2 à environ 5,4, et dans laquelle la dérive de pH de la formulation est de moins d'environ 0,3 unité de pH après un stockage pendant au moins environ 6 mois à température ambiante.

2. Formulation selon la revendication 1, dans laquelle le sulfate d'éphédrine est présent à une concentration d'environ 5 mg/ml.

3. Formulation selon la revendication 1, dans laquelle l'agent de tonicité comprend du chlorure de sodium, du dextrose, du mannitol, du tréhalose, du chlorure de potassium, du glycérol, ou une combinaison de ceux-ci.

4. Formulation selon la revendication 3, dans laquelle l'agent tonique est présent dans une quantité suffisante pour conférer à la formulation une pression osmotique d'environ 310 à 330 mOsm/kg.

5. Formulation selon la revendication 1, dans laquelle la formulation est sensiblement exempte d'un ajusteur de pH qui est séparé ou distinct du tampon citrate.

6. Formulation selon la revendication 1, dans laquelle la formulation a une dérive de pH de moins d'environ 0,2 unité de pH après un stockage pendant au moins environ 6 mois à température ambiante.

7. Formulation selon la revendication 1, dans laquelle la formulation ne contient pas plus d'environ 0,2 % de toute impureté individuelle et pas plus d'environ 2 % d'impuretés totales telles que déterminées par une méthode de pourcentage de surface de pic par chromatographie liquide à haute performance (CLHP) après un stockage pendant au moins environ 6 mois à température ambiante.

8. Formulation selon la revendication 1, dans laquelle la formulation a une coloration de pas moins de B8, telle que déterminée par la Méthode 2.2.2 de la Ph. Eur. après un stockage pendant au moins environ 6 mois à température ambiante.

9. Seringue contenant la formulation selon la revendication 1, dans laquelle la cartouche de seringue comprend un copolymère d'oléfine cyclique et le piston comprend un caoutchouc bromobutyle non enrobé.

10. Formulation selon la revendication 1 comprenant environ 5 mg/ml de sulfate d'éphédrine, l'agent tonique présent dans une quantité suffisante pour conférer à la formulation une pression osmotique d'environ 310 à 330 mOsm/kg, le tampon citrate présent dans une quantité suffisante pour conférer à la formulation le pH allant d'environ 5,2 à environ 5,4, dans laquelle le tampon est le tampon citrate présent à une concentration d'environ 4 à 10 mM.

11. Formulation selon la revendication 10, dans laquelle la formulation est sensiblement exempte d'un ajusteur de pH qui est séparé ou distinct du tampon citrate.

12. Formulation selon la revendication 10, dans laquelle la formulation a une dérive de pH de moins d'environ 0,2 unité de pH après un stockage pendant au moins environ 6 mois à température ambiante.

13. Formulation selon la revendication 10, dans laquelle la formulation ne contient pas plus d'environ 0,2 % de toute impureté individuelle et pas plus d'environ 2 % d'impuretés totales telles que déterminées par une méthode de pourcentage de surface de pic par chromatographie liquide à haute performance (CLHP) après un stockage pendant au moins environ 6 mois à température ambiante.

14. Formulation selon la revendication 11, dans laquelle la formulation a une coloration de pas moins de B8, telle que déterminée par la Méthode 2.2.2 de la Ph. Eur. après un stockage pendant au moins environ 6 mois à température ambiante.

15. Seringue contenant la formulation selon la revendication 10, dans laquelle la cartouche de seringue comprend un copolymère d'oléfine cyclique et le piston comprend un caoutchouc bromobutyle non enrobé.
